# EUROPEAN PATENT SPECIFICATION

(11) **EP 0 810 291 B1**
(45) Date of publication and mention of the grant of the patent: **29.10.2003**
(21) Application number: 96901985.0
(22) Date of filing: 13.02.1996
(51) Int. Cl.: C12Q 1/68, C07H 21/04, G01N 33/50, C12N 15/00

(54) **PROBE FOR USE IN NUCLEIC ACID ANALYSIS AND DETECTING METHOD BASED ON EXCIMER FLUORESCENCE**
SONDE ZUR ANWENDUNG IN DER ANALYSE VON NUKLEINSAÜREN UND VERFAHREN DER DETEKTION BASIEREND AUF EXCIMER-FLUORESZENZ
SONDE DESTINEE A L'ANALYSE DE L'ACIDE NUCLEIQUE ET PROCEDE DE DETECTION A FLUORESCENCE D'EXCIMERE

(30) Priority: 17.02.1995 JP 2958195; 26.10.1995 JP 27908995
(43) Date of publication of application: 03.12.1997
(73) Proprietor: Hamamatsu Photonics K.K., Shizuoka-ken 435-8558 (JP)
(72) Inventor: MASUKO, Masayuki, Hamamatsu-shi Shizuoka-ken 435 (JP); EBATA, Katsuyoshi, Chiba-shi, Chiba 263 (JP)
(74) Representative: West, Alan Harry
(86) International application number: JP9600297
(87) International publication number: WO96025518

(56) References cited:
- WO-A-92/14845
- WO-A-93/09128
- JP-A- 5 211 872
- JP-A- 62 157 570
- US-A- 5 332 659
- US-A- 5 466 578
- CARDULLO R A ET AL: "DETECTION OF NUCLEIC ACID HYBRIDIZATION BY NONRADIATIVE FLUORESCENCE RESONANCE ENERGY TRANSFER" PROCEEDINGS OF THE NATIONAL ACADEMY OF SCIENCES OF USA, NATIONAL ACADEMY OF SCIENCE. WASHINGTON, US, vol. 85, no. 23, 1 December 1988 (1988-12-01), pages 8790-8794, XP000453537 ISSN: 0027-8424
- Photophysics of Aromatic Molecules, Ed. J.Birks, Wiley - Interscience, (1970), pages 301-371

## Description

### TECHNICAL FIELD

This invention relates to radioisotope-free probes for nucleic acid analysis capable of recognizing base sequences with high sensitivity and high selectivity, and to a method of detection using the probes.

### BACKGROUND ART

In genetic engineering, a phenomenon called nucleic acid hybridization is used to detect a particular gene or nucleic acid from a cell or virus. Many hybridization methods have been developed and then utilized depending on purposes to be attained, kinds of genes to be targeted, and ways of detection.

Various methods have also been developed for the identification and quantitation of hybridization products. Generally, these methods use a polynucleotide as a probe, which hybridizes to a complementary nucleic acid to be detected, and requires the partial labeling of the polynucleotide. For example, one of the most sensitive methods commonly used is a method using a radioisotope. Non-radioactive labeling techniques include labeling with a fluorescent substance, an enzyme and biotin, and the substances that interact each other.

Most foregoing conventional hybridization methods use a single detection probe labeled with substances as above. And such probe-is a polynucleotide complementary to a targeted nucleic acid (hereafter called target or target polynucleotide). The labeled probe is used for hybridization in excess over the amount of its target. Before detection of the hybrid so formed, the excess probe polynucleotide must be removed by washing or electrophoresis and so on. This procedure is complicated and time-consuming.

Recently, there has been an intense demand for the development of a detection method which should have practical sensitivity (detection limit) performance and high recognition (capable of recognizing a difference by one base) performance without using any radioisotope. The method should also utilize a phenomenon measurable without the aid of washing out the excess probe after hybridization. Some methods are already known to partially satisfy these requirements. For example, U.S.P. 5,332,659 discloses a method which enables detection without requiring the removal of the excess probe. A phenomenon to be detected in this method is based on the formation of an excimer of two chromophores as labeling groups. In this method, a single probe for detection of a polynucleotide, having two or more fluorescent labeling groups in the probe in advance, is hybridized to a target nucleic acid. A change in the intensity of an excimer fluorescence upon the hybridization is observed, thereby detecting the target nucleic acid.

There may be a case in which a long target nucleic acid is detected by hybridization using an oligonucleotide as a probe. To permit, in such a case, a high recognition of a difference by one base (point mutation recognition) while minimizing a recognition error, an object of the present invention, it is clearly preferred to use a set of two or more probes for a single target, rather than a single probe.

### DISCLOSURE OF THE INVENTION

A method based on this aspect is known which measures a phenomenon occurring when a plural number of probes conjugated with labeling groups are hybridized to their single target nucleic acid, thereby to detect the target nucleic acid.. The labeling groups are a set of energy donor (D) and an energy acceptor (A), which simultaneously bind to different oligonucleotides. A different set of labeling groups for energy transfer is known, but has one component enzyme-labeled. EP0229943A2 discloses a labeling method of which the detection is based on D/A. Its spectral change on the energy transfer from the donor to the acceptor are essentially not sensitive to the locations of the D and A. Furthermore, the fluorescence spectra of the donor and the acceptor overlap markedly. This makes the exact recognition of the difference by one base a very difficult task. The optimal spacing between the fluorophores is 2-7 nucleotide units. EP0070685B1 discloses labeling with an enzyme which is essentially thermally unstable. This method is unsuitable for hybridization that often requires heat treatment. In this method, moreover, the energy donor is a luminescent substance, such as luminol, which is not present in the probe, but is incorporated in an assay solution as a substrate for the enzyme label. This substance diffuses in the solution, and thus is not so sensitive as to be able to specify the distance between the enzyme and the acceptor. Therefore, this method is unsuitable for hybridization which is required to precisely recognize the difference by one base.

The present invention has been accomplished in the light of the above-described problems with conventional methods. It employs a nucleic acid hybridization label which is non-radioactive, as well as provides a highly sensitive method capable of recognizing a difference by one base. The invention can also specifically detect a complex of the nucleic acid with the probe without washing out the excess probe added to an assay solution.

That is, the kit relevant to the present invention comprises a set of two or more oligonucleotide probes as defined in claim 1 that completely hybridize to the complementary consecutive base sequence of the target nucleic acid, the adjacent terminals (i.e. the 3'-terminal of one probe and the 5'-terminal of the other probe) of the probes being labeled with a chromophore group capable of being in the suitable spatial configuration so that an excimer can be formed when probes hybridize simultaneously to the target nucleic acid.

The above-mentioned composition of the invented probe is unpredictable from the known conventional methods, as will be discussed below. Nor can the effects resulting from the construction be expected at all. In detail, the invented probe comprises a set of a plurality of labeled probes designed to be able to recognize even a difference corresponding to one base. Moreover, a phenomenon taking place in between the labeling groups to be detected is such that an excimer fluorescence is highly effectively induced upon hybridization, although the labeling groups are present on the different probes. This fluorescence can be easily differentiated from the monomer emission. In other words, a single chromophore on each different probe is in a spatial configuration such that only when hybridized to the target nucleic acid, does the chromophore efficiently generate the excimer fluorescence together with a chromophore on an adjacent probe. Based on the excimer fluorescence, the target nucleic acid can be detected with very high recognizability because the excimer fluorescence is remarkably red-shifted with respect to the corresponding monomer emission bands. These facts are utterly unexpectable.

The foregoing construction of the present invention, and the effects of the invention based thereon will be described below.

### (1) Erroneous recognition

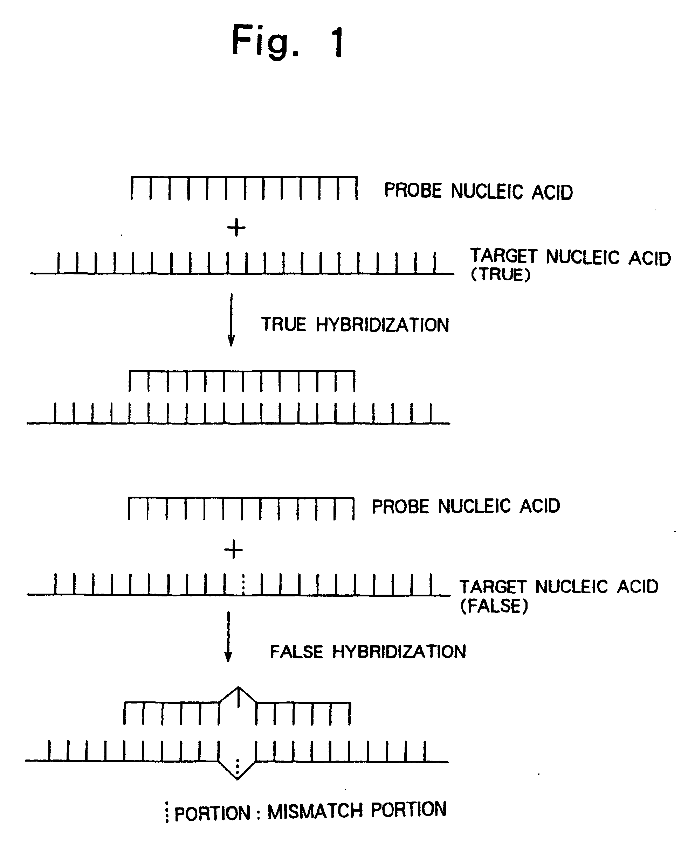

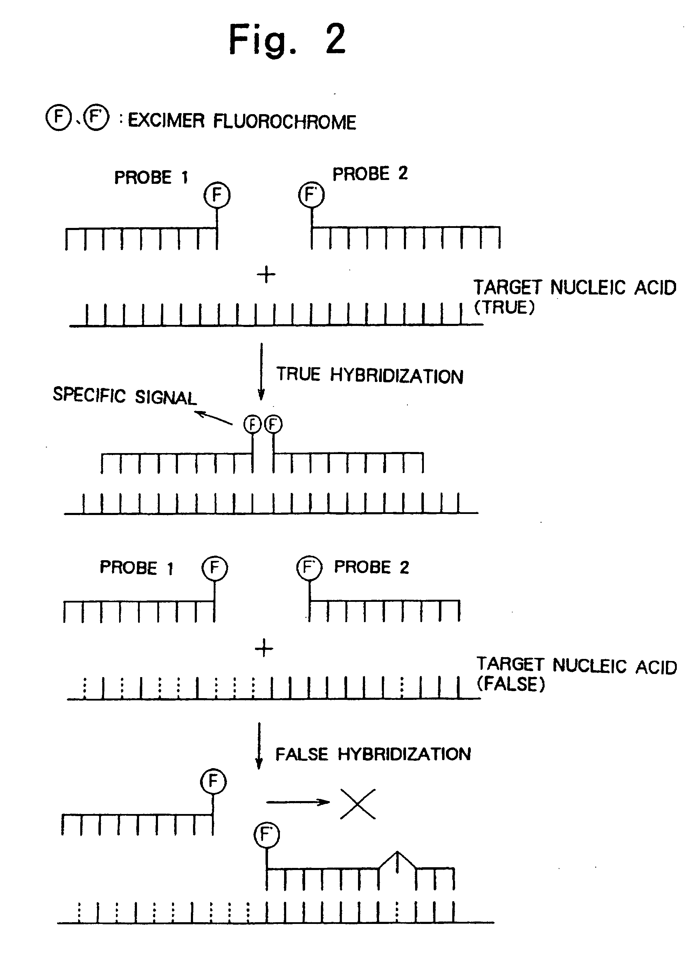

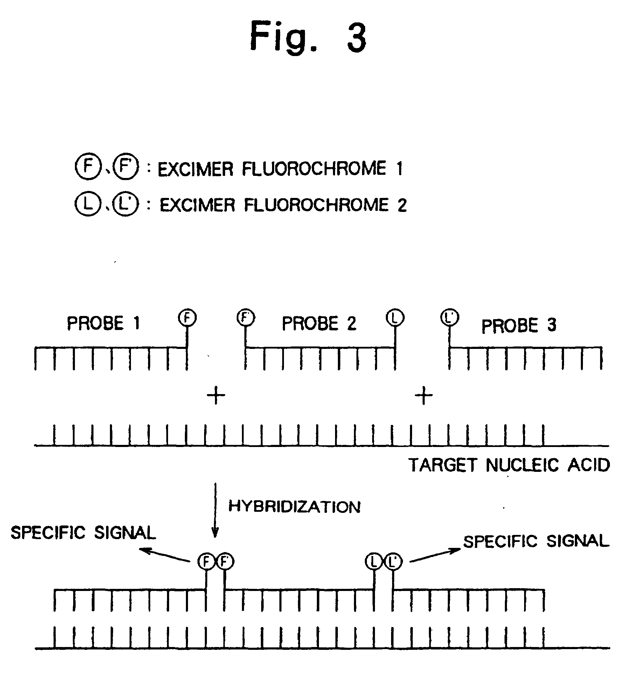

In the detection of a target nucleic acid by the known hybridization methods, there may be a case in which the probe is only one and the target nucleic acid is long. In such a case, hybridization can occur between the probe and a false nucleic acid which is complementary to the target nucleic acid partially or to some extent (False Hybridization in Fig. 1). This type of hybridization results in erroneous recognition. A way of avoiding such a common problem is to use a set of two or more probes rather than a single probe (Fig. 2), and takes advantage of the fact that only when the two probes properly hybridize to a target nucleic acid (True Hybridization in Fig. 2), a specific phenomenon occurs between the two labeling groups. The specific phenomenon does not occur with erroneous recognition (False Hybridization in Fig. 2). This method is not necessarily restricted to using two probes, but may, if necessary, concurrently use more than two probes (Fig. 3). In this case, right recognition is achieved only when specific phenomena emerging between the adjacent labeling groups can all be observed simultaneously. Thus, erroneous recognition can be remarkably avoided.

### (2) Wide variety of labeling groups

The labeling groups that can be used should be as many as possible. Furthermore, the labeling group should not preclude the probe from hybridizing to the target nucleic acid. It must also be avoided for the labeling group to be intercalated into the duplex of nucleic acid base by a hydrophobic interaction with bases. Making a molecular design which satisfy these requirements is considerably difficult in the known prior art having two or more known labeling groups at an intermediate part of a single-stranded probe. The probe of the present invention that comprises a set of a plurality of different probes, on the other hand, can be relieved of those restrictions. That is, it suffices to provide, additionally, one labeling group at the terminus of each probe. Thus, a variety of conventional linking can be available for the present invention. Furthermore, it is possible to widely select the specific phenomena to be measured. It is possible to easily predesign an optimal chemical structure for measurement of the phenomena. For example, the chemical structure of the linker arm between a chromophore and a terminal nucleotide can be chosen, from various possibilities the length of the linker can be optimized, and the chemical stability, temperature stability and storage stability of the labeling group can be controlled.

### (3) Detection sensitivity

A general requirement for hybridization methods is that a background noise must be minimized to increase detection sensitivity. When one probe with a labeling substance is used, the unhybridized probe itself also inherently causes the phenomenon to be measured. The degree to which this occurs varies with the degree of hybridization. This fact constitutes a basis for detection. To reduce the noise, therefore, it is desirable that the specific phenomenon can be observed only when the right hybridization to the target nucleic acid takes place. In this respect, preference is given to the method of emitting specific fluorescence accompanied with hybridization using a set of plural probes.

In the method using a set of plural probes, step-by-step hybridizations can be performed for each probe. Thereby, the impurities, the unhybridized target nucleic acid, and the unhybridized excess probes can be washed out to decrease the background noise to the limit.

### (4) Recognition of the difference by one base

If it is desired to discriminate a sequences of nucleic acid which are different from each other by only one base at a particular position (one base mutation), such specific detection is difficult by using a single-stranded probe. Detection of the one-base difference is possible, on the other hand, with a method in which a set of two probes undergo hybridization so as to sandwich the mutated nucleic acid base, and then a specific phenomenon occurs only when the adjacent terminal labeling groups come in close proximity. Excimer fluorescence, known to identify a specific phenomenon at the level of several angstroms, is preferred for the detection of base difference. The utilization of the excimer fluorescence phenomenon enables a spatial difference (several angstroms) as small as one base to be recognized sensitively.

When the energy transfer (A/D) is used or the enzyme reaction or chemiluminescence is utilized, by contrast, the extent to which the difference can be recognized spatially is much larger than that of the excimer method, reaching several tens of to several hundred angstroms.

Hence, the present invention uses excimer formation for detection that can recognize a difference of one base, one of the objects of the present invention.

Thus, it is clear that the present method, which comprises a set of two probes each having a labeling group and can produce a specific phenomenon only upon proper hybridization, markedly overcome the disadvantages with the conventional methods.

### BRIEF DESCRIPTION OF THE DRAWINGS

Fig. 1 is a view showing the hybridization of a single-stranded nucleic acid detection probe to a true target nucleic acid or a false target nucleic acid, illustrating that erroneous recognition can occur upon false hybridization;
Fig. 2 is a view showing the hybridization of nucleic acid detection probes related to the present invention, which comprises a set of two probes, and a true target nucleic acid or a false target nucleic acid, illustrating that no erroneous recognition occurs upon hybridization of one of the constituent probes to the false target nucleic acid;
Fig. 3 shows an embodiment in which a very long target nucleic acid is detected with nucleic acid detection probes, which comprises a set of three or more probes, for minimizing the possibility of erroneous recognition;
Fig. 4 is a view showing changes in the fluorescence spectra of solutions containing various concentrations of a target oligonucleotide and two kinds of the detection probe;
Fig. 5 is a view showing changes in the relative excimer fluorescence intensities (at 495 nm) in the fluorescence spectra of solutions containing various concentrations of a target oligonucleotide and two kinds of the detection probe;
Fig. 6 is a view showing the effects of the length of the linker arms between a pyrene residue (label) and a terminal sugar moiety on the excimer formation between a target 32-mer and the couple of pyrene butanoic acid-induced 16-mer probe (common) and one of the following pyrenealkyliodoacetamide-introduced 16-mer probes: PIA = N-(1-pyrene)iodoacetamide; PMIA = N-(1-pyrenemethyl)iodoacetamide; PEIA = N-(1-pyreneethyl)iodoacetamide; and PPIA = N-(1-pyrenepropyl)iodoacetamide;
Fig. 7 shows that a target nucleic acid containing an unhybridizable sequence as a result of point mutation, that make the labels apart, and that this can be detected with the probe of the present invention, in which pyrenemethyl iodoacetamide-introduced 16-mer and pyrene butyric acid hydrazide-introduced 16-mer are used, and 32-mer target has a continuous sequence;
Fig. 8 shows that excimer fluorescence remarkably decreases when the two probes are made apart from each other for the insertion of a sequence forming no duplex, demonstrating that the present invention can be used for detection of a point mutated nucleic acid;
Fig. 9 is a view showing the structure of Compound 4;
Fig. 10 is a view showing the structure of Compound 5; and
Fig. 11 is a view showing the structure of Compound 6.

### BEST MODE FOR CARRYING OUT THE INVENTION

A nucleic acid analysis kit of the present invention is a couple of probes as defined in claim 1, i.e., probe 1 and probe 2, for detecting a single target polynucleotide having a sequence of the number q of bases, and the base sequence of the first nucleic acid analysis probe, probe 1, is complementary to a sequence of the number r of bases consecutive from the 5'-terminal of the target polynucleotide (r = an integer of 1 or more, but (q-1) or less) and has a chromogenic group molecule at the 5'-terminal of the probe 1 via a linker arm, while the base sequence of the second nucleic acid analysis probe, probe 2, is complementary to a sequence of the bases between the (r+1)th base and the qth base from the 5'-terminal of the target polynucleotide having the sequence of the number q of bases and has a chromogenic group at the 3'-terminal of the probe 2 via a linker arm. The number of probes in one set should not be limited to two, but may be more than two. Even in the latter case, the basic construction comprises a set of two probes, and the actions and effects based on this construction are the same. Thus, the following description will take a set of two probes as an example.

The invented nucleic acid analysis probes are characterized by forming an excimer and emitting fluorescence in a longer wavelength region than those of the chromogenic group of the probe 1 and the chromogenic group of the probe 2 when these probes simultaneously hybridize to the target polynucleotide.

The invented nucleic acid analysis probes are also characterized in that the fluorescence is ascribed to an excimer formed from the chromogenic group of the probe 1 and the chromogenic group of the probe 2 when these probes simultaneously hybridize to the target polynucleotide.

The invented nucleic acid analysis probes may contain a chromogenic group selected from the group consisting of pyrene, naphthalene, anthracene, perylene, stilbene, benzene, toluene, phenylanthracene, diphenylanthracene, benzpyrene, benzanthracene, tetracene, phenanthrene, pentacene, triphenylene, and chrysene.

The chromogenic group may be pyrene.

The length of the linker arm between the 5'-terminal nucleotide of the nucleic acid analysis probe 1 and the chromogenic group and the length of the linker arm between the 3'-terminal nucleotide of the nucleic acid analysis probe 2 and the chromogenic group may be between 3 angstroms and 20 angstroms, preferably between 5 angstroms and 20 angstroms.

The linker arm between the 5'-terminal nucleotide of the nucleic acid analysis probe 1 and the chromogenic group, or the linker arm between the 3'-terminal nucleotide of the nucleic acid analysis probe 2 and the chromogenic group may be a substituent of the formula -(CH₂)n-(X)k-(CH₂)m-Y- where X is selected from the group consisting of CONH, NHCO, COO, OCO, O, S and NH, Y is selected from the group consisting of O, S, NH and (PO₃⁻)S, and n and m denote an integer of 0 to 5, and k denotes 0 or 1.

The method of nucleic acid detection according to the present invention detects a polynucleotide having a sequence of the number q of bases by its hybridization to two probes, i.e., probe 1 and probe 2 by a method as defined in claim 7. This method comprises a step of mixing the target polynucleotide, the first nucleic acid analysis probe, probe 1, and the second nucleic acid analysis probe, probe 2, and the probe 1 is complementary to a sequence of the number r of bases consecutive from the 5'-terminal of the target polynucleotide (r = an integer of 1 or more, but (q-1) or less), having a chromogenic group at the 5'-terminal of the probe 1 via a linker arm, and the probe 2 is complementary to a sequence of the bases between the (r+1)th base and the qth base from the 5'-terminal of the target polynucleotide, having a chromogenic group at the 3'-terminal of the probe 2 via a linker arm; and a step of measuring fluorescence in a longer wavelength region than those of the chromogenic group of the probe 1 and the chromogenic group of the probe 2 after the mixing. More specifically, the invented nucleic acid detection probes are polynucleotides which strictly recognize the base sequence of the target polynucleotide by hybridizing to the target polynucleotide, the object of detection.

Furthermore, the two invented probe polynucleotides have an excimer -forming chromogenic group, such as pyrene, at the 5'-terminus and the 3'-terminus, respectively. Upon the hybridization of the target polynucleotide to the polynucleotides of the invented probes, the chromogenic groups come in close proximity. As the monomer emission from the chromogenic groups decreases, the excimer emission increases in a longer wavelength region.

When the two chromogenic groups take a spatially favorable configuration for excimer, a potent excimer can be formed upon irradiation. As the monomer emission from the chromogenic groups markedly decreases, strong excimer fluorescence can be observed in a longer wavelength region.

The target polynucleotide can be identified and detected by measuring the excimer fluorescence red-shifted. The identification and detection also become possible by measurement of decrease in monomer fluorescence intensity.

The preferred embodiments of the present invention will now be described.

### [Target nucleic acid (target polynucleotide)]

The target nucleic acids that can be detected with the invented nucleic acid detection probes are not restricted. They are those which can usually be hybridized by known methods. Preferred examples include DNA, RNA (tRNA, mRNA, rRNA), synthetic oligonucleotide, synthetic polynucleotide, synthetic deoxyoligonucleotide, synthetic deoxypolynucleotide, and a heteropolymer of deoxyribonucleotide and ribonucleotide. The base sequence of the target nucleic acid must be previously identified. Based on this base sequence information, a probe completely complementary to this nucleic acid will be prepared and hybridized.

The way of knowing the base sequence of the target nucleic acid for the above purpose may be a known method of base sequence determination (e.g. the Sanger method (dideoxy-mediated chain-termination method for DNA sequencing)).

### [Nucleic acid detection probe]

Polynucleotide of the invented nucleic acid detection probe comprises a set of two polynucleotides which completely hybridize to the complementary portions of the target polynucleotide, the object of detection.

That is, the two polynucleotides relevant to the present invention have their respective base sequences so that probe 1 and probe 2 simultaneously hybridize to the base sequence of the target polynucleotide, as shown in Fig. 2.

An arbitrary number of bases from the 5'-terminal of the target nucleic acid can be determined on the basis of sequence of the probe 1. Accordingly, the base sequence of the probe 2 will be automatically determined.

If one of the probes has too small the number of bases, however, the probe cannot hybridize to the complementary target nucleotide, not identifying the target sequence. The preferred number of bases falls within a certain range, so that the number of the bases in the base sequence of the probe 2 or probe 1 should desirably be 8 or more.

The method of synthesizing the base sequence necessary for the probe 1 or 2 is not restricted to the present invention. It may be an ordinary nucleotide modification method (e.g., the method described in Handbook of Fluorescent Probes and Research Chemicals, 5th ed, 1992-1994, by R.P. Haugland, Molecular Probes, Inc.), or automatic synthesis (e.g., the method described in Oligonucleotides and Analogues, A Practical Approach, ed. By F. Eckstein, IRL Press). Naturally occurring oligonucleotide may also be used after a chromophore is labeled.

The resulting polynucleotide can be purified by, say, reversed phase high performance liquid chromatography.

### [Linker Arm]

The polynucleotides of the two probes related to the present invention are labeled with a labeling group (chromogenic group molecule, chromophore, fluorophore) through a chain linker at the 5'- and 3'-terminal of the probes.

When these two probe polynucleotides are hybridized to the target polynucleotide, they are arranged so that the two chromogenic groups from the respective probe polynucleotide can be brought spatially close to each other with the aid of linker arms.

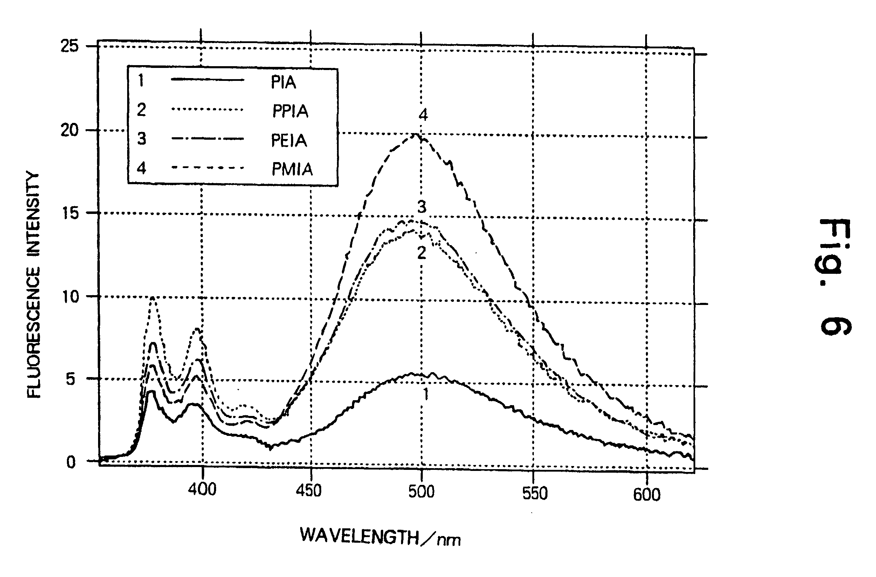

Thus, the length of the linker arm between the 5'-terminal or 3'-terminal and the chromogenic group is of utmost importance in the present invention, and can be adjusted depending on the type and length of the linker. In the'present invention, in particular, the inventors' findings have clarified that the length of the linker greatly affects the detection sensitivity. Fig. 6 shows that excimer formation based on pyrene-pyrene interaction is markedly influenced by the length of linkers of the probes. In the present invention, therefore, the length of the linker is a measure for optimizing a detection method. Concretely, the length of the chain linker refers to the sum of the lengths based on the number of bonds, provided that the single covalent bonds, such as C-C, C-O, C-N, N-N, C-S and P-O, from the terminal nucleotide of the probe to the chromogenic group are the same length (1.4 angstroms).

For example, when the 5'-carbon of the 5'-terminal nucleotide is labeled with the chromogenic group via a chain linker of the formula -O-P-S-CH₂-CONH-CH₂- as in (5'-C)-O-P-S-CH₂-CONH-CH₂-chromogenic group molecule, the length of the chain linker is 1.4x8 = 11.2 angstroms.

Also, when the 3'-carbon of the 3'-terminal nucleotide is labeled with the chromogenic group via a chain substituent (linker) of the formula -NH-NH-CO-CH₂-CH₂-CH₂-CH₂- as in (3'-C)-NH-NH-CO-CH₂-CH₂-CH₂-CH₂-chromogenic group molecule, the length of the chain substituent is 1.4x8 = 11.2 angstroms.

As already shown in Fig. 6, in order for the chromogenic group molecules to be spatially close to each other, producing an excimer, the linkers, whether too short or too long, may result in an ineffective spatial configuration.

According to the inventors' findings, when the chromogenic group was a pyrenyl group, it was preferably located in a space at least 3 angstroms, more preferably, not less than 5 angstroms but not more than 20 angstroms, apart from the 5'- or 3'-carbon of the terminal nucleotide.

If the chain linker is 3 angstroms or less long, the chromogenic group molecules will be unable to approach each other. In this case, the formation of an excimer or the like, or excimer fluorescence or the like will not be able to occur substantially.

Investigation using the CPK molecular model has proved effective, suggesting that the length of the chain linker is preferably not less than 3 angstroms, but more preferably, not more than 20 angstroms. A still more preferred length is 5 angstroms or more, but 15 angstroms or less. In more detail, the inventors have found, based on the measurement of excimer fluorescence, that when a pyrenyl group is the chromogenic group, 5 to 10 angstroms is a preferred range. In other words, conjugation of a pyrenylmethyl group to the 5'-or 3'-carbon via an ether linkage (4.2 angstroms) is not preferred.

In the present invention, there are no restrictions for the type and the method of synthesis of the linker, as far as the above length of the linker is attained. A preferred example is a methylene group, an amide group, an ester group, an ether group, a thiophosphoric ester, or a couple of these. Decision of a suitable manner of linker conjugation based on the global consideration of chemical stability, thermal stability and preference of a labeling group is a matter of easy choice for those skilled in the art.

### [Chromogenic group, labeling group, chromophore, fluorophore]

The chromogenic group (or labeling group) usable for the present invention is the one containing a chromophore in a part. Such a chromogenic group is not restricted to a specific group except that it forms excimer. Various chromogenic groups can be introduced to nucleotides by an ordinary method of synthesis.

The chromophore is selected from the group consisting of pyrene, naphthalene, anthracene, perylene, stilbene, benzene, toluene, phenylanthracene, diphenylanthracene, benzpyrene, benzanthracene, tetracene, phenanthrene, pentacene, triphenylene, and chrysene. Preferably usable are aromatic chromophores, and more preferred examples are chromogenic groups having a pyrene ring.

In the present invention, it is preferred that the chromogenic groups in the two probes are the same. That is, the chromogenic groups are not of the donor/acceptor of fluorescence resonance energy transfer.

Hence, the fluorescence shifted to a longer wavelength region according to the present invention is not of the electron transfer type, as will be described later on. That is, the present invention makes it possible to observe excimer fluorescence emitted from the same chromogenic groups. This fluorescence is generated as a result of the spatial approach of the two chromogenic groups due to the hybridization explained previously. Thus, only the probes correctly hybridized to the target nucleic acid can be detected.

Even if the excess unhybridized probe is coexistent, only the hybridized probes can be detected, without interference by monomer fluorescence ascribed to the unhybridize probe.

### [Hybridization]

There is no restriction on the method for hybridizing the target polynucleotide to the polynucleotides of the two nucleic acid detection probes in the present invention.

The ordinary conditions for hybridization can be preferably applied to the present invention (e.g., the method described in Molecular Cloning, A Laboratory manual, 2nd. ed., J. Sambrook et al., Cold Spring Harbor Laboratory Press, 1989).

For example, the two probes and the target nucleic acid can be hybridized preferably by mixing them in a solution at 25°C. Alternatively, they may be hybridized by reacting them at a higher temperature (e.g., a temperature 10°C lower than the melting temperature of the hybrid dissociation), followed by annealing to return them to the room temperature.

In the present invention, moreover, the target polynucleotide can be identified or quantitatively determined without using washing or other means after hybridization.

The method of detection is not restricted, as far as it is a means of measuring fluorescence. For instance, the fluorescence can be measured preferably using a commercially available fluorometer.

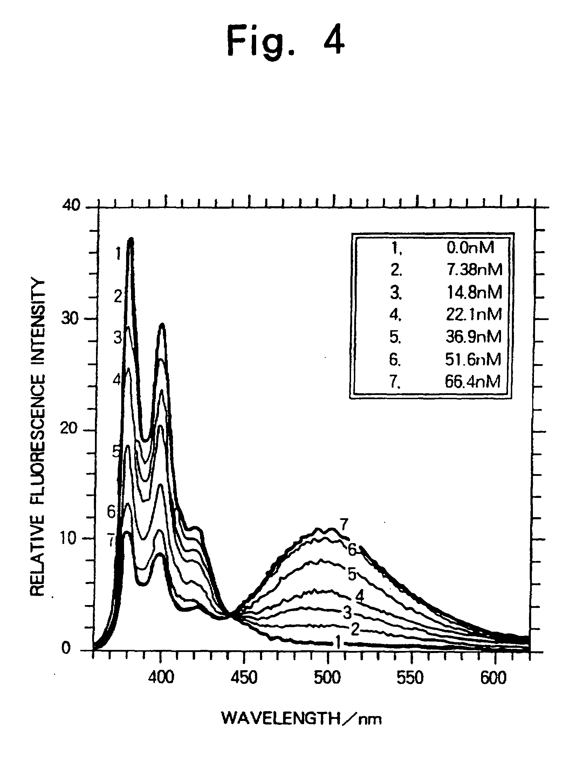

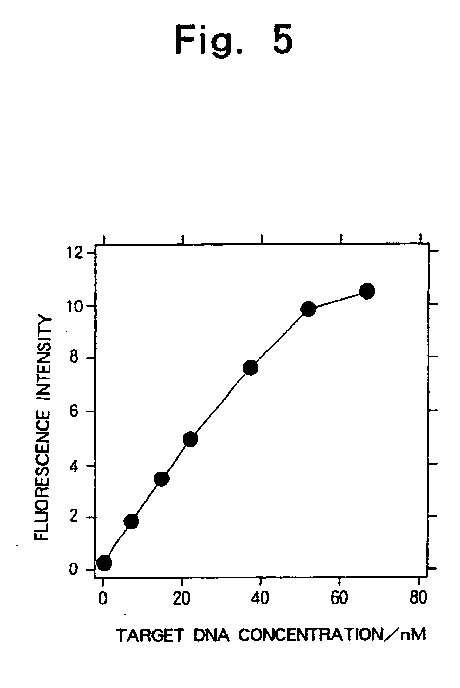

As shown in Fig. 4, it is possible to measure not only the fluorescence of the chromogenic group per se (pyrene in this case)(peak wavelength 380 nm), but also excimer fluorescence shifted to the longer wavelength region (peak wavelength about 500 nm). Needless to say, this excimer peak is not observed at all before hybridization. Thus, quantitative determination of the hybridized target is extremely easy. Drawing of a precise calibration curve enables the target nucleic acid concentration to be determined upon measurement of the fluorescence intensity, as shown in Fig. 5. The experimental data of Fig. 5 obtained using an ordinary fluorometer do not indicate the detection limits of the present invention. An improvement in the sensitivity of the fluorometer would permit detection with higher sensitivity. This optimization of the measurement system is a matter of easy choice for those skilled in the art. However, Fig. 6 shows that at least about several nM can be detected.

Particularly, the analytical probe of the invention may be used in excess, and the above-described procedure can be performed without interference by monomer fluorescence due to the chromogenic group of the excess probe that has not been hybridized.

### [Detection of point-mutated nucleic acid]

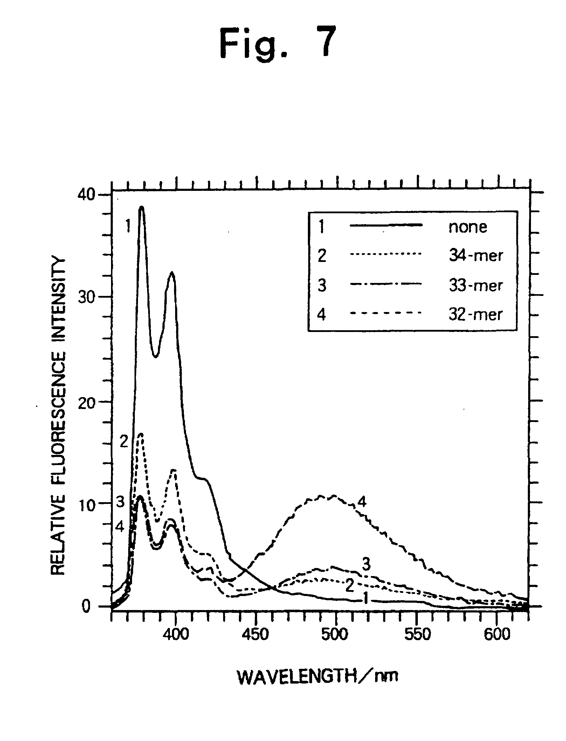

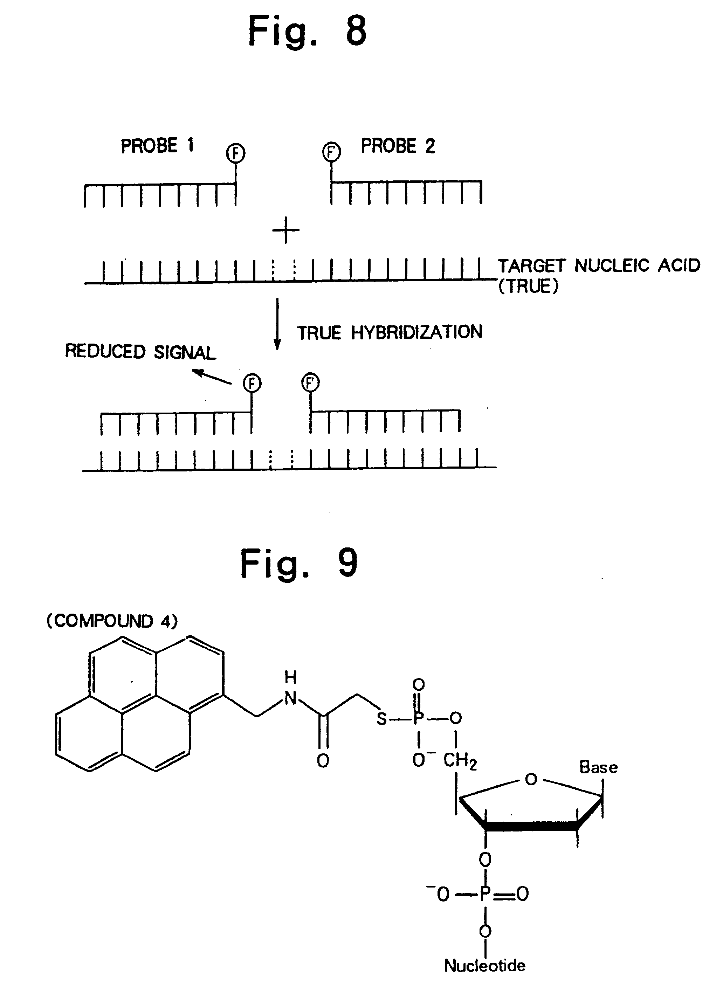

The principle of detection relevant to the present invention is, as explained earlier, that the two probes correctly hybridize to the target nucleic acid to generate strong excimer fluorescence, whose measurement results in the detection and quantitative determination of the target nucleic acid. Therefore, even if there are nucleic acid bases which cannot form a base pair (duplex) between the terminals of the two probes conjugated with the labeling groups, the probes may be designed such that such bases will be removed from the base sequences of the probes. The so designed probes can be used for the highly sensitive, highly selective detection of a target nucleic acid different only in such a base sequence, namely, a point mutated nucleic acid. This possibility is suggested in Fig. 7. In the presence of about one or two nucleic acid bases between the two probes, remarkable decreases in excimer fluorescence intensity occur in the present invention. From these results, point mutation can be identified (Fig. 8).

The present invention will be described in more detail with reference to specific examples, which do not limit the invention.

The 3'-terminal nucleotide of the probe 2 (Compound 3) in the following description was ribonucleotide, except that deoxyribonucleotide was used in other compounds.

### [Synthesis of oligonucleotide]

Oligonucleotide 32-mer selected as a model to be detected had the sequence: 5'-AGAGGGCACGGATACCGCGAGGTGGAGCGAAT-3' (Compound 1) A nucleotide complementary to the base sequence from the 5'-terminal to the 16th base of the target was used as nucleic acid detection probe 1: 3'-TCTCCCGTGCCTATGG-5'          (Compound 2) Further, a nucleotide complementary to the base sequence covering the 17th to 32nd bases from the 5'-terminal of the oligonucleotide 32-mer (Compound 1) was used as nucleic acid detection probe 2: 3'-(C)GCTCCACCTCGCTTA-5' (Compound 3) (however, only the nucleotide with (C) is ribonucleotide). These nucleotides were all synthesized by the solid-phase phosphoramidite technique using an automatic synthesizer (Millipore Limited).

The oligonucleotide 32-mer (Compound 1) and the two probe oligonucleotide 16-mers (Compound 2, Compound 3) were each separated and purified by reversed phase high performance liquid chromatography (column; PepRPCTM 4m HR5/5, Pharmacia Fine Chemicals; elution solvent system, acetonitrile/0.1M ammonium acetate mixed solvent gradient; detection wavelength, 260 nm).

All the compounds were desalted by gel filtration, and freeze-dried for condensation.

### [Introduction of pyrene chromogenic group into the 5'-terminal of the nucleic acid detection probe 1 (3'-TCTCCCGTGCCTATGG-5')(Compound 2)]

Into the probe 1 oligonucleotide (Compound 2, molecular weight 4904.21, extinction coefficient ε 139.9 mmol⁻¹·liter·cm⁻¹) obtained above, a pyrene group was introduced by the method of Czworkowski et al. (Czworkowski J. et al., Biochemistry, 30, page 4821, 1991) in accordance with the following procedure:
(i) Two mg of Compound 2 was mixed with 2.5 ml of a solution comprising 140 mM Tris-HCL (pH 7.6) containing 20 mM MgCl₂ and 0.2M KCl, 2.135 ml of water, 0.05 ml of 500 mM dithiothreitol, 0.5 ml of 100 mM adenosine-5'-O-(3'-thiotriphosphate) (lithium salt, Boehringer Mannheim), and 0.04 ml of a T₄ polynucleotide kinase solution (Takara). The mixture was allowed to stand for 3 hours at 36°C at a lightproof state.
(ii) Further, 2.5 ml of 2M NaCl and 2.5 ml of water were added, and then a 1:1 (volume ratio) chloroform/methanol mixture was added. The mixture was centrifuged to recover the aqueous phase.
(iii) To the resulting aqueous phase, 10 ml of chloroform was added, and the mixture was centrifuged to recover the aqueous phase. After precipitation with ethanol, the resulting deoxyoligonucleotide was concentrated, and solvent exchange was performed using a column of Sephadex G-25 (Pharmacia Fine Chemicals) equilibrated with 25 mM HEPES/NaOH (pH 7.4) containing 0.5 mM NaCl and 10 mM mercaptoethanol.
(iv) After deoxyoligonucleotide was solidified to dryness by ethanol precipitation and centrifugal concentration, the following solvents were added in order; Four ml of 50 mM bicine/KOH (pH 8.4), 5 ml of dimethylformamide (DMF), and 1.0 ml of a DMF solution of (N-(1-pyrenylmethyl)iodoacetamide (Molecular Probe) were added, and the mixture was stirred for 4 hours at room temperature.
(v) The excess reaction mixture was removed by ethanol precipitation, and the residue was centrifugally concentrated to obtain Compound 4. Its structure is shown in Fig. 9.
(vi) For further purification, ODS(Octadecylsilane) reversed-phase high performance liquid chromatography was employed. The column used was a column (21.5 mm x 15 cm) of TSKgel OligoDNA RP (TOSOH).

A mixture of acetonitrile and 0.1M ammonium acetate was used for elution, and the column was eluted with the following concentration gradient (elution rate 1 ml/min, room temperature): 5:95 for the first 5 minutes, changed to 40:60 for a subsequent 175 minutes.
(vii) The resulting sample was centrifugally concentrated at room temperature. The final product was dissolved in 0.01M Tris-HCl buffer (pH 7.5) containing 3.0 mM EDTA and 0.1M NaCl, and stored in frozen state at -85°C.

### [Introduction of pyrene chromogenic group into the 3'-terminal of the nucleic acid detection probe 2 (3'-(C)GCTCCACCTCGCTTA-5')(Compound 3), Part 1]

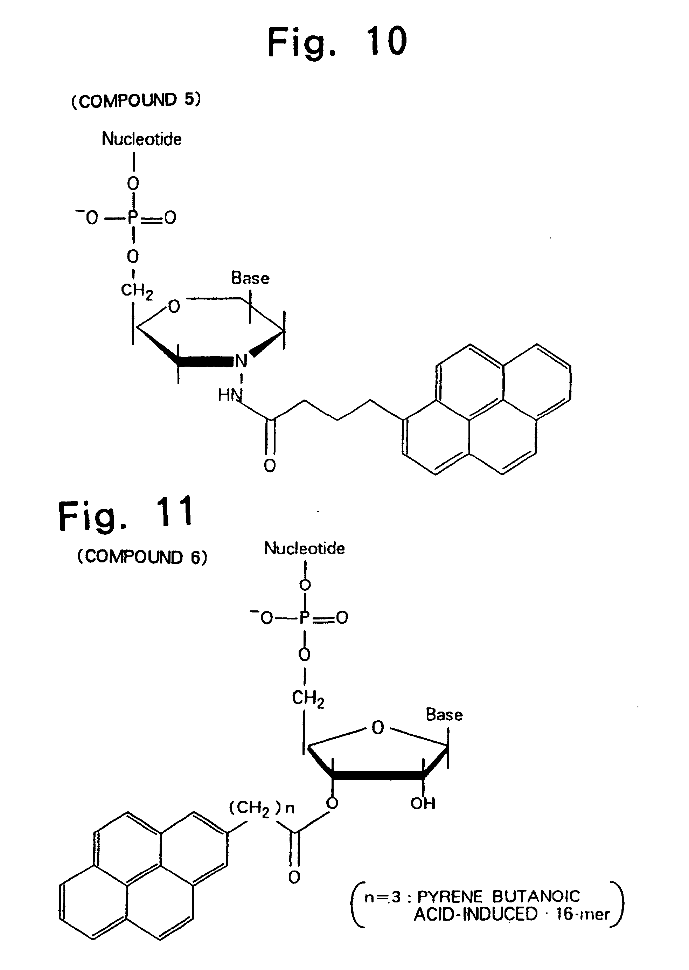

Into the probe 2 oligonucleotide (Compound 3, molecular weight 4849.18, extinction coefficient ε 136.4 mmol⁻¹·liter·cm⁻¹) synthesized according to the method described previously, a pyrene group was introduced by a modified form of the method of Reins, Cantor et al. (Koenig, P., Reins, S.A., Cantor, C.R.(1977), "Pyrene Derivatives as Fluorescent Probes of Conformation Near the 3'-Termini of Polyribonucleotides", Biopolymers 16, 2231-2242) in accordance with the following procedure:
(i) 45.5 Micrograms of the oligonucleotide (Compound 3) was dissolved in 0.5 ml of 0.05M acetate buffer (pH 5.6).
(ii) 2.0 Milliliters of 0.05M acetate buffer (pH 5.6) containing 110 mg NaIO₄ and 7.5M urea was added, and the mixture was allowed to stand for 45 minutes at room temperature under lightproof conditions.
(iii) Further, 0.5 ml of KCl was added, and the mixture was left to stand at 4°C to precipitate the excess NaIO₄.
(iv) After the precipitate was removed by centrifugation, oligonucleotide was precipitated by ethanol precipitation.
(v) The precipitate was dissolved in a small volume of 0.05M acetate buffer (pH 5.6) containing 3mM EDTA, whereafter the solution was desalted through a Sephadex G-25 column equilibrated with the same buffer.
(vi) The oligonucleotide fractions were collected with the absorbance at 280 nm as a measure. They were combined and concentrated by ethanol precipitation.
(vii) To the precipitate, 3.0 ml of 0.05M acetate buffer (pH 5.6) was added, followed by adding 3.0 ml of dimethyl sulfoxide (DMSO) having 3 mg of 1-pyrenebutyric acid hydrazide dissolved therein. The mixture was allowed to stand for 2 hours at 37°C under protection from light.
(viii) Further, 0.3 ml of 2M KCl was added, and then an excess of ethanol was added to precipitate oligonucleotide.
(ix) After centrifugation, 1.0 ml of 0.01M Tris-HCl buffer (pH 7.5) containing 3.0 mM EDTA sodium salt and 0.1M KCl was added to the precipitate to redissolve it.
(x) Finally, NaBH₃CN (sodium cyanoborohydride) was added, and the reduction reaction was performed for 1 hour at room temperature. The amount of NaBH₃CN added was about 320 mols per mol of the probe.
(xi) Then, ethanol precipitation was performed several times until confirmation of the complete disappearance of fluorescence emitted from pyrene in the supernatant after centrifugation. Then, the final precipitate was solidified to dryness by centrifugal evaporation to obtain Compound 5. Fig. 10 shows the structure of Compound 5
(xii) Purification was performed by ODS reversed phase high performance liquid chromatography. The column used was a column (21.5 mm x 15 cm) of TSKgel OligoDNA RP (TOSOH). The solvent used was a gradient by a mixture of acetonitrile and 0.1M ammonium acetate.
(xiii) The resulting Compound 5 was centrifugally concentrated at room temperature. The product was dissolved in 0.01M Tris-HCl buffer (pH 7.6) containing 3.0 mM EDTA sodium salt and 0.1M NaCl, and stored in frozen state at -85°C.

### [Introduction of pyrene chromogenic group into the 3'-terminal of the nucleic acid detection probe 2 (3'-(C)GCTCCACCTCGCTTA-5')(Compound 3), Part 2]

To introduce a pyrene chromogenic group into the 3'-terminal of the nucleic acid detection probe 2 (Compound 3), the method described below was also used. This method was a partially modified form of the method of B.P. Gottikh et al. (Gottikh, B.P., Krayevsky, A.A., Tarussova, N.B., Tsilevich, T.L., Tetrahedron, 26, 4419-4433(1970)), and it is herein called the CDI (carbonyldiimidazole) method.
(i) A 1.2M CDI solution in DMF(dimethylformamide) was prepared.
(ii) To 0.1 ml of this solution, 0.1 ml of a DMF solution of 0.4M 1-pyrenebutanoic acid was added, and the mixture was stirred for about 30 minutes at room temperature.
(iii) To the resulting solution, an aqueous solution of the 16-mer in a concentration of 400 nnmol/0.5 ml was added, and the mixture was incubated for 3 hours at room temperature under dark conditions.
(iv) The solvent was removed by means of centrifugal evaporation.
(v) To the residue, water and chloroform were added in turn for solvent extraction.
(vi) The aqueous fraction was collected, and chloroform was added again for solvent extraction.
(vii) The solvent was removed from the collected aqueous fraction by pipetting and then condensated by centrifugal evaporation.
(viii) Purification was performed by adding a small amount of water, and subjecting the mixture to an ODS reversed-phase high performance liquid chromatograph. The column used was a 21.5 mm x 15 cm column of TSKgel OligoDNA RP (TOSOH). Elution was carried out using a mixture of acetonitrile and 0.1M ammonium acetate with the following concentration gradient (elution rate 1 ml/min, room temperature): 5:95 for the first 5 minutes, changed to 40:60 for a subsequent period of 175 minutes.
(ix) The resulting sample (pyrenebutanoic acid-introduced 16-mer) (Compound 6) was dissolved in 0.1M phosphate buffer (pH 7), and stored at -85°C. Fig. 11 shows the structure of Compound 6.

### [Basic procedure for hybridization]

About 66 nM of the target oligonucleotide (Compound 1) and about 66 nM of the probe oligonucleotides (Compounds 4, Compound 5) were added to 10 mM phosphate buffer (pH 7.0) containing 20% (v/v) dimethylformamide (DMF) and 0.2M NaCl. The mixture was incubated at 25°C. By monitoring the absorbance (A260) of the nucleic acid with respect to the temperature of this solution, i.e., the hyperchromic effect, hybridization was found to be complete when the temperature was 25°C or lower.

### [Confirmation of excimer formation]

Fluorescence spectra were measured under the above-mentioned basic hybridization conditions. As shown in Fig. 4, fluorescence characteristic of the pyrene monomer was observed in a wavelength region around 400 nm, and at the same time, a broad fluorescence band was observed at about 500 nm. Such a broad fluorescence band at about 500 nm is observed only in the presence of the three oligonucleotides described above, and resembles the fluorescence spectra of known pyrene excimers. Thus, that fluorescence band is attributable to excimer fluorescence (Birks, J.B., Christophorous, L.G.(1963), Spectrochim. Acta 19, 401-410).

If the above fluorescence band at 495 nm is due to excimer fluorescence, its intensities should be accompanied by decreases in the intensity of the pyrene monomer fluorescence. Actually, as the concentration of the target oligonucleotide increased, the intensity ratio of excimer to monomer was found to increase (Fig. 4).

These findings led to the conclusion that the fluorescence band at about 500 nm was excimer fluorescence.

### [Possibility for quantitative analysis of target nucleic acid]

Fig. 5 shows the relative intensities of the fluorescence band at about 500 nm obtained when various concentrations of the target oligonucleotide were added to a solution containing equal amounts of the nucleic acid detection probes (Compound 4, Compound 5) each present in excess. The use of the calibration curve shown in Fig. 5 enables the target oligonucleotide to be determined quantitatively.

### [Dependence of excimer fluorescence intensity on the length of a linker arm]

Fig. 6 shows the fluorescence spectra of hybrids consisting of the target nucleic acid 32-mer, and equimolar concentrations of the pyrenebutyric acid-introduced 16-mer probe and the pyrenealkyl iodoacetamide-introduced 16-mer probe. The pyrenealkyl iodoacetamide indicated here is different from pyrenemethyl iodoacetamide in the length of the linker arm (namely, the alkyl chain portion). However, the method of its synthesis is exactly the same as for pyrenemethyl iodoacetamide-introduced 16-mer probe.

As shown in Fig. 6, even when one of the probes is identical (pyrenebutanoic acid-introduced 16-mer), a change in the length of the linker arm of the other probe by merely one methylene group exerts a significant influence on the quantum yield of excimer fluorescence. Under these experimental conditions, the pyrenemethyl iodoacetamide-introduced 16-mer shows the highest quantum yield, and the linker longer or shorter than that of the pyrenemethyl iodoacetamide-introduced 16-mer produces poorer results.

From these facts, a length of 11.4 angstroms from the 5'-carbon of the terminal deoxyribose is assumed to be the optimal length of the linker. The length of the linker arm is 9.8 angstroms for PIA, and 12.6 angstroms for PPIA.

### [Detection of point mutated target nucleic acid]

The inventors have further investigated hybridization in which the base sequence of the aforementioned two probes of the invention are not completely continuous for the targeted sequence. As shown in Fig. 7, when the two probes were 1 or 2 nucleotides apart from each other on the hybrid after the hybridization(Fig. 8), excimer formation was markedly suppressed. This finding demonstrates that the continuous arrangement of the two probes on the hybrid is prerequisite for intense excimer emission.

These facts mean that, when the hybridization is designed for adjacent terminal ends of two probes to be apart by distance corresponding to one- or two-base sequence (point-mutated region, see Fig.8) from each other on the hybrid, a wild type gene and a point-mutated gene can be distinguished and identified in a homogeneous solution.

In our model experiments (Fig.7), the target nucleic acid is the aforementioned sequence (namely 33-mer or 34-mer) having one or two thymine deoxyribonucleotides inserted into the middle of the 32-mer. Thus, there may be a great distance between the adjacent terminals (5'-terminal of one probe and 3'-terminal of the other probe) on the hybrid upon hybridization (see Fig. 8). As a result, an optimum configuration for excimer formation may not be achieved.

### Industrial Applicability

The present invention provides nucleic acid detection probes which are non-radioactive and based on hybridization, and provides a highly sensitive method capable of recognizing a one-base difference among nucleic acid sequences. The invention can also specifically detect a complex of the target nucleic acid and the probe without washing out the excess probe added to a reaction mixture after the hybridization reaction.

## Claims

1. A kit for nucleic acid analysis, comprising a first probe 1 and a second probe 2 for detecting a target polynucleotide having a sequence of q bases, the first probe 1 having a base sequence complementary to a sequence of r consecutive bases from the 5'-terminal of the target polynucleotide, r being an integer of between 1 and (q-1), and having a chromogenic group linked to the 5'-terminal nucleotide via a linker arm, and the second probe 2 having a base sequence complementary to a sequence between the (r+1) base and the q base from the 5'-terminal of the target polynucleotide, and having a chromogenic group linked to the 3'-terminal nucleotide via a linker arm, such that when the probes 1 and 2 hybridize to the target polynucleotide the chromogenic group of probe 1 and the chromogenic group of probe 2 are adjacent and form an excimer which fluorescences at a longer wavelength than the chromogenic group of probe 1 or the chromogenic group of probe 2.

2. A kit according to claim 1, wherein the chromogenic group linked to probe 1 or to probe 2 is selected from pyrene, naphthalene, anthracene, perylene, stilbene, benzene, toluene, phenylanthracene, diphenylanthracene, benzpyrene, benzanthracene, tetracene, phenanthrene, pentacene, triphenylene and chrysene.

3. A kit according to claim 2, wherein the chromogenic group is pyrene.

4. A kit according to any one of claims 1 to 3, wherein the length of the linker arm linked to probe 1 or to probe 2 is 0,3 to 2 nm (3 to 20 Å).

5. A kit according to claim 4, wherein the length of the linker arm is 0,5 to 2 nm (5 to 20 Å).

6. A kit according to any one of claims 1 to 5, wherein the linker arm linked to probe 1 or the linker arm linked to probe 2 is of the formula
-(CH₂)n-(X)k-(CH₂)m-Y-
in which X is selected from CONH, NHCO, COO, OCO, O, S and NH; Y is selected from O, S, NH and (PO₃⁻)S; each of n and m is zero or an integer from 1 to 5; and k is zero or 1.

7. A method for detecting a target polynucleotide having a sequence of q bases by hybridization to first and second nucleic acid probes 1 and 2, the method including the steps of mixing the target polynucleotide with first and second nucleic acid probes 1 and 2 as defined in any one of claims 1 to 6, allowing the probes to hybridize to the target polynucleotide, and measuring the fluorescence thereof at a longer wavelength than that at which the chromogenic groups of the probes fluoresce.

## Patentansprüche

1. Kit für die Nukleinsäureanalyse, welcher eine erste Sonde 1 und eine zweite Sonde 2 zum Nachweisen eines Zielpolynukleotids einer Sequenz aus q Basen enthält, wobei die Sonde 1 eine Basensequenz, die einer Sequenz aus r aufeinander folgenden Basen vom 5'-terminalen Ende des Zielpolynukleotids komplementär ist, wobei r eine ganze Zahl zwischen 1 und (q-1) ist, und eine chromogene Gruppe hat, die mit dem 5'-terminalen Nukleotid über einen Linkerarm verknüpft ist, und wobei die zweite Sonde 2 eine Basensequenz, die einer Sequenz zwischen der Base (r+1) und der Base q vom 5'-terminalen Ende des Zielpolynukleotids komplementär ist, und eine chromogene Gruppe hat, die mit dem 3'-terminalen Nukleotid über einen Linkerarm verknüpft ist, so daß, wenn die Sonden 1 und 2 an das Zielpolynukleotid hybridisieren, die chromogene Gruppe der Sonde 1 und die chromogene Gruppe der Sonde 2 benachbart sind und ein Excimer bilden, das bei einer längeren Wellenlänge fluoresziert als die chromogene Gruppe der Sonde 1 und die chromogene Gruppe der Sonde 2.

2. Kit nach Anspruch 1, worin die chromogene Gruppe, die mit der Sonde 1 oder der Sonde 2 verknüpft ist, unter Pyrin, Naphthalin, Anthracen, Perylen, Stilben, Benzol, Toluol, Phenylanthracen, Diphenylanthracen, Benzpyren, Benzanthracen, Tetracen, Phenanthren, Pentacen, Triphenylen und Chrysen ausgewählt ist.

3. Kit nach Anspruch 2, worin die chromogene Gruppe Pyren ist.

4. Kit nach einem der Ansprüche 1 bis 3 , wobei die Länge des Linkerarms, der mit der Sonde 1 oder der Sonde 2 verknüpft ist, 0,3 bis 2 nm (3 bis 20 Å) ist.

5. Kit nach Anspruch 4, wobei die Länge des Linkerarms 0,5 bis 2 nm (5 bis 20 Å) ist.

6. Kit nach einem der Ansprüche 1 bis 5, wobei der Linkerarm, der mit der Sonde 1 verknüpft ist, oder der Linkerarm, der mit der Sonde 2 verknüpft ist, die Formel
-(CH₂)n-(X)k-(CH₂)m-Y-
hat, worin X unter CONH, NHCO, COO, OCO, O, S und NH ausgewählt ist, Y unter O, S, NH und (PO₃⁻)S ausgewählt ist, n und m jeweils 0 oder eine ganze Zahl von 1 bis 5 sind und k 0 oder 1 ist.

7. Verfahren zum Nachweisen eines Zielpolynukleotids mit einer Sequenz aus q Basen durch Hybridisieren an erste und zweite Nukleinsäuresonden 1 und 2, wobei das Verfahren die Stufen umfaßt, bei denen das Zielpolynukleotid mit den ersten und zweiten Nukleinsäureproben 1 und 2 nach einem der Ansprüche 1 bis 6 gemischt wird, die Proben an das Zielpolynukleotid hybridisiert werden und deren Fluoreszenz bei einer längeren Wellenlänge als derjenigen, bei der die chromogenen Gruppen der Sonden fluoreszieren, gemessen wird.

## Revendications

1. Kit pour l'analyse d'acide nucléique, comprenant une première sonde 1 et une seconde sonde 2 pour détecter un polynucléotide cible ayant une séquence de q bases, la première sonde 1 ayant une séquence de bases complémentaire à une séquence de r bases consécutives à partir de l'extrémité 5'-terminale du polynucléotide cible, r étant un nombre entier entre 1 et (q-1), et ayant un groupe chromogène lié à un nucléotide 5'-terminal via un bras lieur, et la seconde sonde 2 ayant une séquence de bases complémentaire à une séquence entre la base (r + 1) et la base q de l'extrémité 5'-terminale du polynucléotide cible, et ayant un groupe chromogène lié au nucléotide 3'-terminal via un bras lieur, tel que quand les sondes 1 et 2 s'hybrident au polynucléotide cible, le groupe chromogène de la sonde 1 et le groupe chromogène de la sonde 2 sont adjacents et forment un excimère qui fluoresce à une longueur d'onde plus longue que celle du groupe chromogène de la sonde 1 ou du groupe chromogène de la sonde 2.

2. Kit selon la revendication 1, dans lequel le groupe chromogène lié à la sonde 1 ou à la sonde 2 est choisi parmi le pyrène, le naphtalène, l'anthracène, le pérylène, le stilbène, le benzène, le toluène, le phénylanthracène, le diphénylanthracène, le benzpyrène, le benzanthracène, le tétracène, le phénanthrène, le pentacène, le triphénylène et le chrysène.

3. Kit selon la revendication 2, dans lequel le groupe chromogène est le pyrène.

4. Kit selon l'une quelconque des revendications 1 à 3, dans lequel la longueur du bras lieur lié à la sonde 1 ou à la sonde 2 est de 0,3 à 2 nm (3 à 20 angströms).

5. Kit selon la revendication 4, dans lequel la longueur du bras lieur est de 0,5 à 2 nm (5 à 20 angströms).

6. Kit selon l'une quelconque des revendications 1 à 5, dans lequel le bras lieur lié à la sonde 1 ou le bras lieur lié à la sonde 2 est de formule
- (CH₂)n-(X)k-(CH₂)m-Y-
dans laquelle X est choisi parmi CONH, NHCO, COO, OCO, O, S et NH ; Y est choisi parmi O, S, NH et (PO₃ )S ; chacun parmi n et m vaut zéro ou un nombre entier de 1 à 5 ; et k vaut zéro ou 1.

7. Procédé pour détecter un polynucléotide ayant une séquence de q bases par hybridation avec les première et seconde sondes d'acide nucléique 1 et 2, le procédé comprenant les étapes de mélange du polynucléotide cible avec la première et la seconde sondes d'acide nucléique 1 et 2 telles que définies selon l'une quelconque des revendications 1 à 6, permettant aux sondes de s'hybrider au polynucléotide cible, et de mesurer la fluorescence de celui-ci à une longueur d'onde plus longue que celle à laquelle les groupes chromogènes des sondes fluorescent.
